# EUROPEAN PATENT SPECIFICATION

(11) **EP 1 219 300 B1**
(45) Date of publication and mention of the grant of the patent: **06.12.2006**
(21) Application number: 00128661.6
(22) Date of filing: 28.12.2000
(51) Int. Cl.: A61K 39/35, A61K 39/36, A61P 37/08

(54) **Treatment of allergies**
Behandlung von Allergien
Traitement des allergies

(43) Date of publication of application: 03.07.2002
(73) Proprietor: BIOMAY Produktions- und Handels- Aktiengesellschaft, 1090 Wien (AT)
(72) Inventor: Mahler, Vera, Dr., 91054 Erlangen (DE); Vrtala, Susanne, Dr., 1210 Wien (AT); Suck, Roland, Dr., 22303 Hamburg (DE); Cromwell, Oliver, Dr., 21465 Wentorf (DE); Fiebig, Helmut, Dr., 21493 Schwarzenbek (DE); Kraft, Dietrich, Dr., 1170 Wien (AT); Valenta, Rudolf, Dr., 2604 Theresienfeld (AT)
(74) Representative: Keller, Günter

(56) References cited:
- WO-A-99/16467
- S. VRTALA ET AL.: "T CELL EPITOPE-CONTAINING HYPOALLERGENIC RECOMBINANT FRAGMENTS OF THE MAJOR BIRCH POLLEN ALLERGEN, Bet v 1, INDUCE BLOCKING ANTIBODIES." THE JOURNAL OF IMMUNOLOGY, vol. 165, no. 11, 1 December 2000 (2000-12-01), pages 6653-6659, XP002179174 BALTIMORE, US

## Description

Type I allergy, a genetically determined hypersensitivity disease, affects almost 25% of the population. The symptoms of Type I allergy which are caused by the production of IgE antibodies against *per se* harmless antigens range from allergic rhinoconjunctivitis, bronchial asthma, dermatitis, gastroenterological problems to anaphylactic shock. Specific immunotherapy, the only curative approach towards allergy treatment, is based on the administration of increasing doses of the disease-eliciting allergens in order to induce a state of "unresponsiveness" towards the applied allergens. Although the clinical efficacy of specific immunotherapy has been documented in numerous controlled clinical studies, it has been difficult to investigate the underlying immunomechanisms. This may be attributed in part to the fact that both treatment of allergic diseases and analysis of clinical and immunological effects have been performed with natural allergen extracts consisting of mixtures of allergenic and non-allergenic components which are difficult to standardize.

A major disadvantage of specific immunotherapy is the risk of inducing life-threatening anaphylactic side effects due to the administration of allergens. Reduction of anaphylactic side effects and simultaneous preservation of immunogenic properties of therapeutic allergen preparations therefore has been a long sought goal. One way to increase the immunogenicity of a therapeutic allergen preparation and to delay systemic release from the site of application is adsorption of allergen extracts to adjuvants (Sledge, R. F., US Naval Med. Bull. 1938, 36: 18, Mellerup, M. T. et al., Clin. Exp. Allergy 2000, 30: 1423-1429). Alternatively chemical modification of allergen extracts by conjugation with monomethoxypolyethylene glycol (mPEG) or treatment with glutaraldehyde or formaldehyde also reduces allergenic activity (Lee, W. Y. and Sehon, A. H., Nature 1977, 267: 618-619, Marsh, D. G. et al., Immunol. 1970, 18: 705-722, Metzger, W. J. et al., J. Allergy Clin. Immunol., 1981, 68: 442-448).

It is an objective of the present invention to provide means for the treatment or prevention of allergic disorders.

Unexpectedly it has been found by the inventors that for the induction of protective, blocking antibodies it is possible to reduce the number of injections and to increase the time interval between the single administrations when using high doses of modified hypoallergenic derivatives of the wildtype allergenic proteins. The present invention covers therefore also methods of treatment of humans and animals in such countries wherein those types of claims are allowable.

The invention therefore relates to the use of a derivative of an allergenic protein which derivative has a reduced allergenic activity compared with an allergenic protein from which it is derived for the preparation of a medicament for the treatment or prevention of an allergic disorder wherein the medicament is administered to a patient four times with the proviso that the time intervals between the administrations are at least 14 days.

The allergenic protein may be any known allergenic protein, e.g. an allergenic protein from a plant allergen source. Allergenic proteins that can be envisaged are preferably Bet V 1, the major grass pollen (e.g. group 1, group 2, group 5 etc.) mite (e.g., Der p 2), bee venom (e.g. phospholipase) and animal hair dander allergens (e.g., Cat: Fel d 1) or oligomers thereof. The derivative used may be any derivative of a wild type allergenic protein. For example the derivative may be a fragment of the wildtype protein or an oligomere thereof. The derivative may also be a peptide or a molecule which results from site directed mutagenesis. It is also possible to chemically modify the naturally occurring wildtype allergenic protein.

The derivative which is used according to the invention may be prepared in different ways. It is preferred however that the derivative is prepared by expression of recombinant DNA in a host cell or by chemical synthesis. When using the first method a polynucleotide encoding the derivative is provided and introduced into a host cell. The host cell may be a procaryotic cell such as E. coli or a eucaryotic cell like yeast. Upon transfection or transformation of the respective cell the cell is cultured under conditions such that the desired derivative is expressed. Eventually, the expression product is recovered from the cell by methods known in the art.

When using the latter method also known methods for peptide synthesis such as solid phase synthesis can be used.

According to the invention the medicament is administered to a patient four times.

It is essential for the present invention that the time intervals between the single administrations are at least 14 days. Preferably, the time intervals are at least 16 days, more preferably at least 18 days, most preferably at least 20 days. It is possible that the time intervals between the single administrations do not vary. However, it is preferred, that the time interval between the last but one application and the last application of the medicament is longer than the preceding time interval(s). In the most preferred embodiment the medicament is administered to a patient four times with the time interval between the third and the fourth administration being longer than the time interval between the first and the second, and the second and the third administration, respectively.

In a preferred embodiment during each administration substantially the same dose of the derivative contained in the medicament is administered to the patient. The dose may be at least 5 microgram of the derivative, preferably at least 10 microgram, most preferably at least 20 micrograms.

It is also preferred that the medicament further contains an adjuvant such as Al(OH)₃ or another compound or composition which stimulates the immune response.

The derivative of an allergenic protein which is used according to the invention has a reduced allergenic activity compared with the respective allergenic wildtype protein from which it is derived. Within the present application the allergenic activity of a protein or a sample is determined in the following way:

The allergenic activity of a sample is determined by determining the IgE antibodies which are induced in a test animal upon application of the sample. The allergenic activity is preferably defined in suitable in vitro or in vivo tests. A preferred in vitro test is the basophil histamine release assay as described in Vrtala et al., J. Clin. Invest. 1997, 99, pp. 1673-1681. Alternatively the allergenic activity is determined in a skin test as described in van Hage-Hamsten et al. J. Allergy Clin. Immunol. 1999, 104, pp. 969-977 or in Pauli et al. Clin. Exp. Allergy 2000, 30, pp. 1076-1084.

Preferably the allergenic activity of the derivative is less than 50% of the allergenic activity of the respective wildtype protein from which it is derived. More preferably the allergenic activity of the derivative is less than 25% of the wildtype protein. In the most preferred embodiment the derivative has substantially no allergenic activity.

It is preferred that the derivative of an allergenic protein leads to induction of IgG antibodies. It is also preferred that the derivative of the allergenic protein upon administration does not elicit a significant IgE response. Most preferably the derivative of an allergenic protein upon administration leads to the induction of IgG antibodies which at least in part are "protective antibodies" i.e. antibodies which prevent IgE antibodies from binding to the respective wildtype protein from which the derivative is derived.

The risk of inducing anaphylactic side effects represents a major disadvantage of allergen-specific immunotherapy (Mellerup, M. T. et al., Clin. Exp. Allergy 2000, 30: 1423-1429).

The vaccine formulations containing modified allergen derivatives usually induce strong IgG₁ and IgG_{2a/b} and IgG₁, IgG₂ and IgG₄ (man) responses to the wildtype allergen when administered in courses of 8 weekly injections with increasing doses followed by one further injection with the maintenance dose after three more weeks. They fail to induce a significant IgE-production.

Notably, it was found that a course of three injections of the maintenance dose given at weekly intervals also induced strong IgG₁ and IgG_{2a/b} responses to the wildtype, but lower IgE responses than the increasing dose scheme. It may thus be feasible to treat patients with a course of only three injections similar to other therapeutic vaccines.

The finding that genetically modified rBet v 1 derivatives induced IgG1 antibodies in mice which crossreacted with the major allergens of alder and hazel pollen indicates that they will not only be useful for treatment of birch pollen-, but also for the treatment of birch-associated allergies (e.g. allergy to alder and hazel pollen). In this context it is noteworthy that clinical immunotherapy studies with birch pollen extract indicated that treatment with birch pollen extract reduced allergic symptoms to pollens of trees of the *Fagales* order and even allergy to apple.

Perhaps more important than the fact that genetically modified rBet v 1 derivatives induced IgG antibodies to rBet v 1 wildtype and to Bet v 1-homologous allergens is the finding that mouse-derived antibodies strongly inhibited the binding of birch pollen allergic patients' IgE to Bet v 1. These results indicate that the genetically modified rBet v 1 derivatives contain sufficient structural elements to induce protective IgG antibodies *in vivo* which either bind to or close to epitopes defined by human IgE antibodies and thus prevent IgE binding to the wildtype allergen. This finding is important in view of the importance of allergen-specific blocking antibodies of the IgG isotype for the successful outcome of specific immunotherapy. In this context, it has been shown that blocking antibodies not only suppress allergen induced effector cell activation but are also effective in inhibiting IgE-mediated allergen presentation to T cells and thus can reduce proliferation and cytokine release from allergen-specific T cells.

One advantage of the new form of vaccination with recombinant allergen derivatives will be the possibility of tailoring treatment to match a patient's sensitivity. Patients will receive a pharmaceutically defined allergen preparation instead of allergen extracts containing a variety of allergenic and non-allergenic components. Another advantage will be that administration of hypoallergenic allergen derivatives will induce less anaphylactic side effects and thus allow treatment with few high dose injections. The latter will favour the induction of a Bet v 1 -specific TH1/Th0 response, the production of blocking IgG antibodies and perhaps of tolerance.

The invention thus represents the basis for clinical immunotherapy trials which study safety and efficacy of the hypoallergenic allergen formulations based on recombinant DNA or synthetic technology and thus for a new generation of rBet v 1 derivative vaccine formulations in birch pollen allergic patients.

### Description of the tables and figures:

Table 1. Inhibition of patients' IgE-antibody binding to rBet v 1 by murine antisera. ELISA-plate bound rBet v 1 was preincubated with a representative serum from a male (m) or a female (f) mouse (increasing-, high dose scheme) which were immunized with fragments, trimer, rBet v 1, or Al(OH)₃, respectively. The percentage inhibition of IgE binding is displayed for 10 sera from birch pollen allergic patients (P1-P10). The right column shows the mean percentage inhibition of IgE binding for the patients.

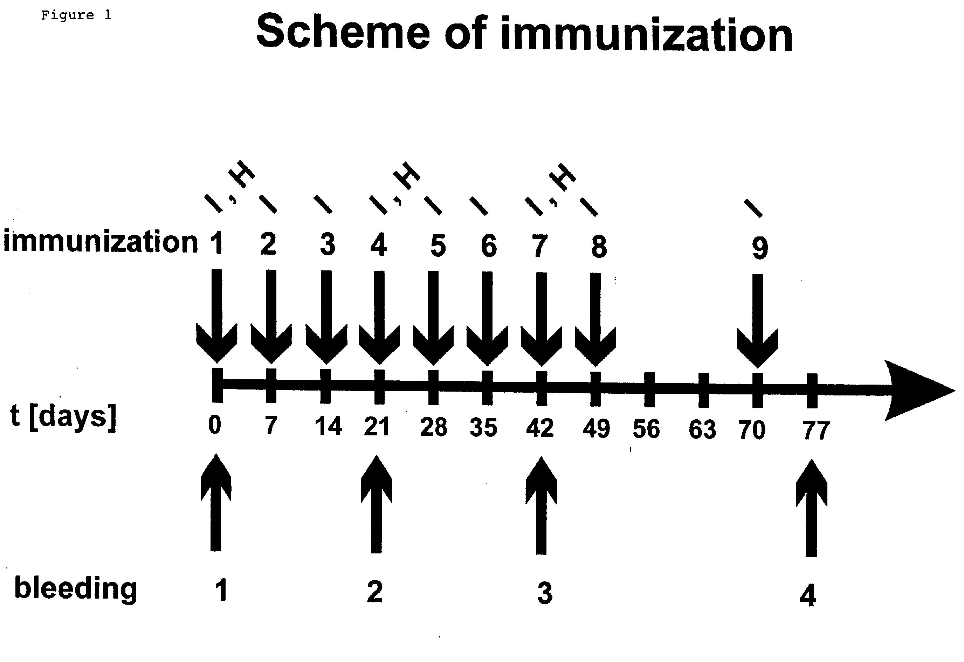

Fig. 1: Scheme of immunization. Groups of mice received either a series of 8 weekly injections containing increasing antigen doses (I) followed by one final injection on day 70 or three high dose immunizations (H) on day 0, 21 and 42. Serum samples were collected from all mice on days 0, 21, 42 and 77.

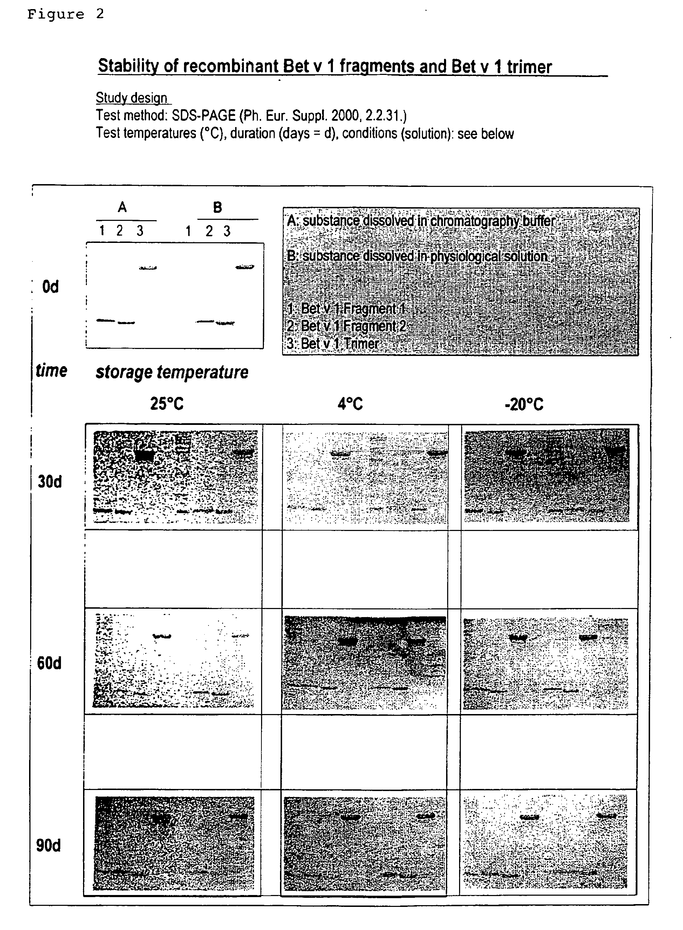

Fig. 2: Commassie-stained gel containing rBet v 1 derivatives after different storage conditions

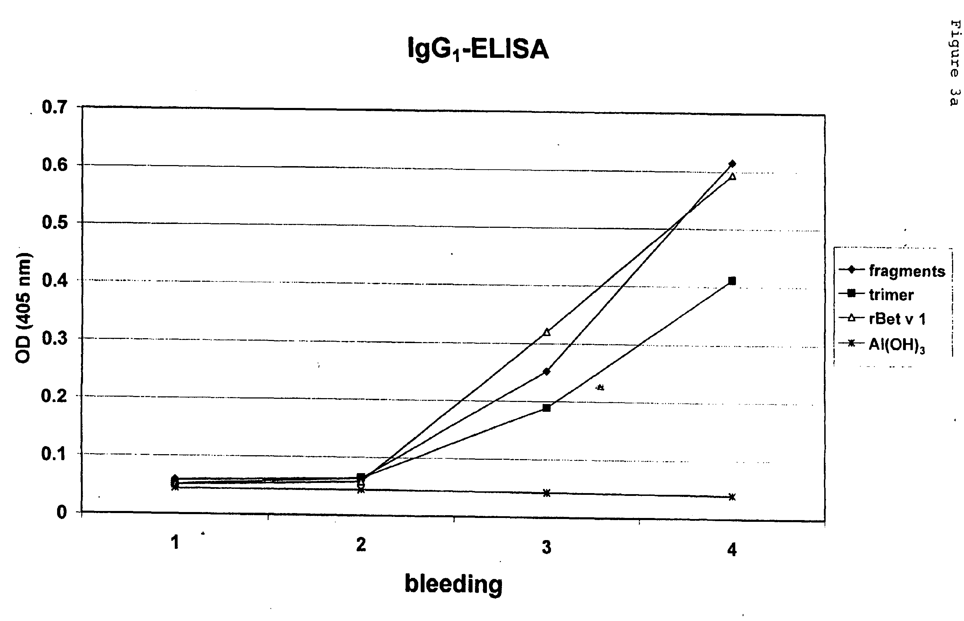

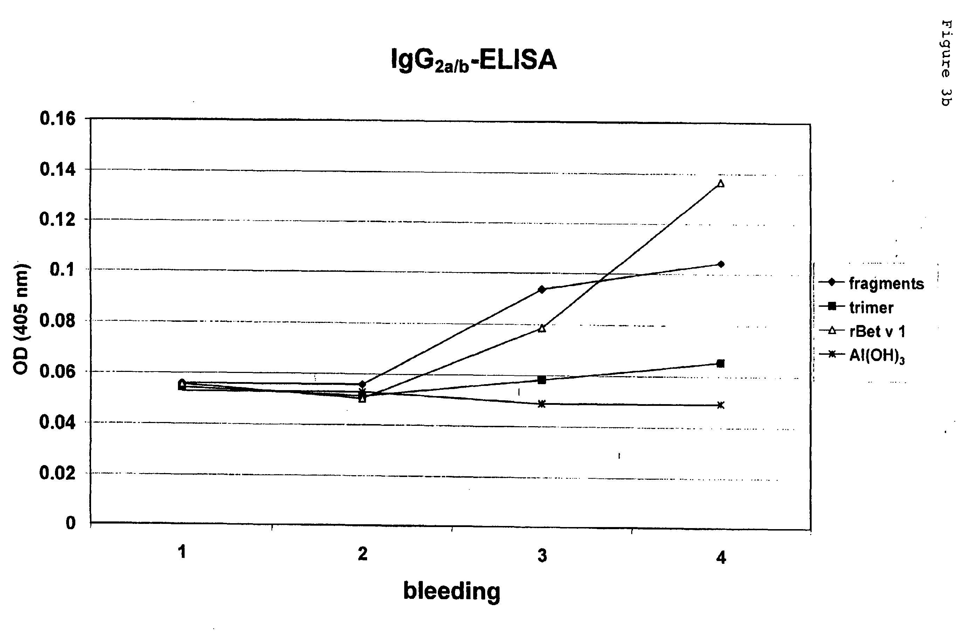

Fig. 3a-c: IgG₁, IgG_{2a/b}, IgE responses to rBet v 1. Groups of 16 mice each were immunized with rBet v 1 fragments, trimer, rBet v 1 wildtype, or Al(OH)₃ (insert) according to an increasing dose scheme. Mean OD values (y-axis) of 16 immunized mice corresponding to the antibody levels are displayed for the different bleedings (x-axis).

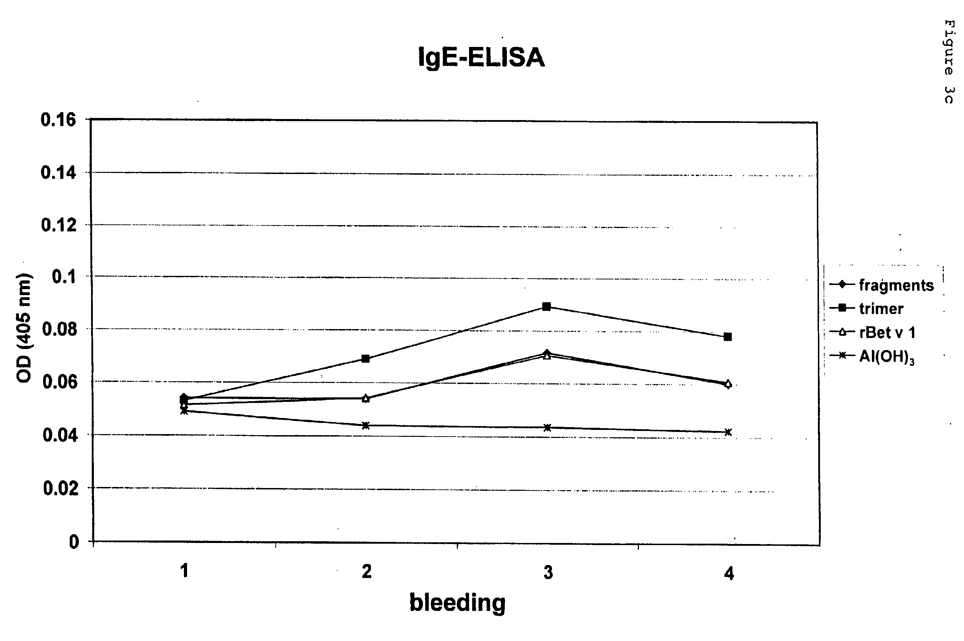

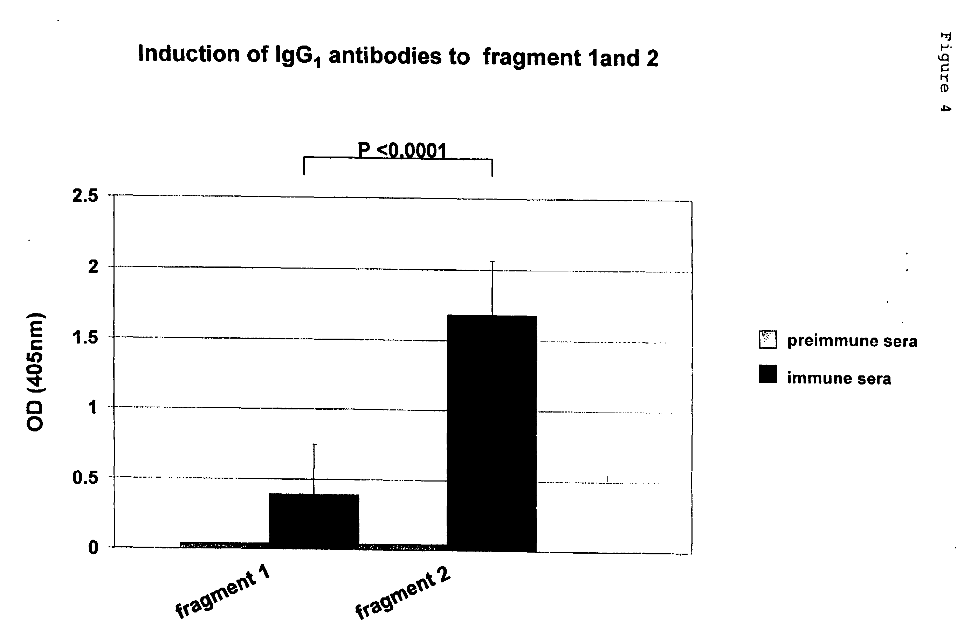

Fig. 4: Induction of IgG₁ to fragment 1 and 2. Mouse sera obtained from mice immunized with the equimolar mixture of rBet v 1 fragments (group I-1) were examined for fragment-specific antibody responses. ELISA plates were coated with rBet v 1-fragment 1 or fragment 2, respectively. Mean OD values (y-axis) corresponding to the IgG₁ reactivity of preimmune and immune sera from 16 mice to the fragments are displayed with the standard deviation.

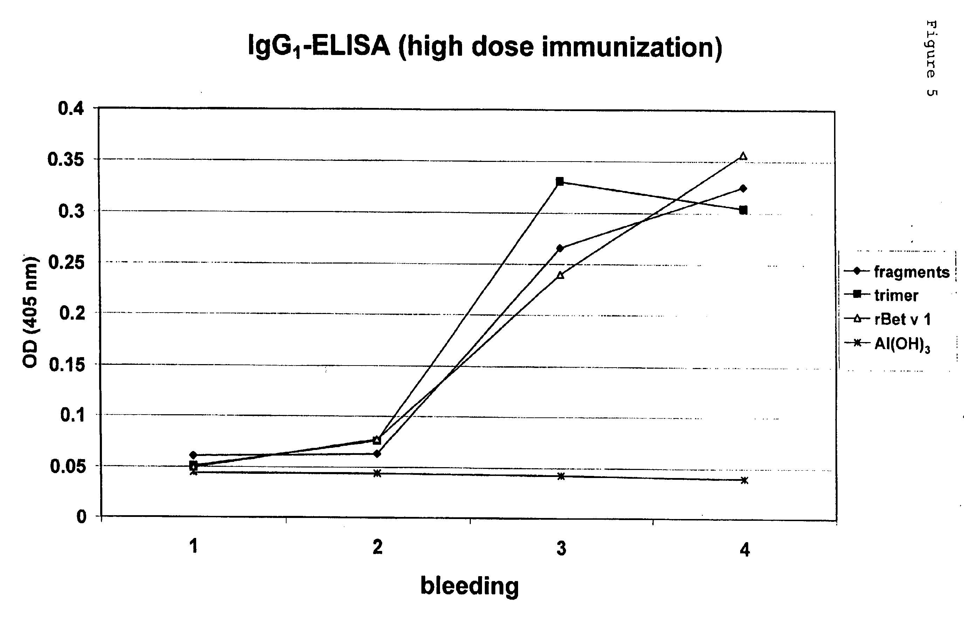

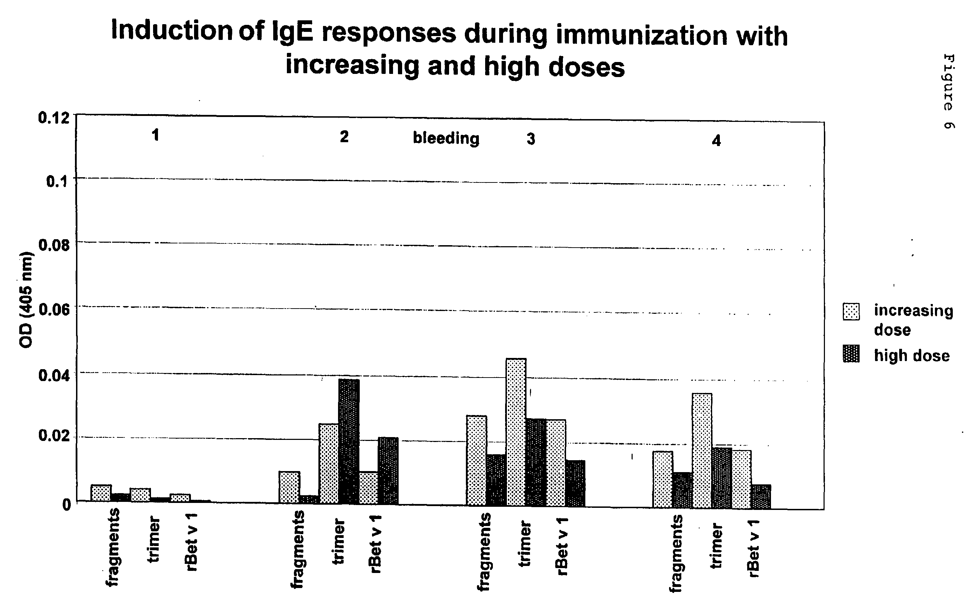

Fig. 5: IgG₁ response to rBet v 1 after immunization according to the high dose scheme. Groups of 16 mice each were immunized with rBet v 1 fragments, trimer, rBet v 1 wildtype, or Al(OH)₃ (insert) according to the high dose scheme. Mean OD values (y-axis) of 16 immunized mice corresponding to the antibody levels are displayed for the different bleedings (x-axis).

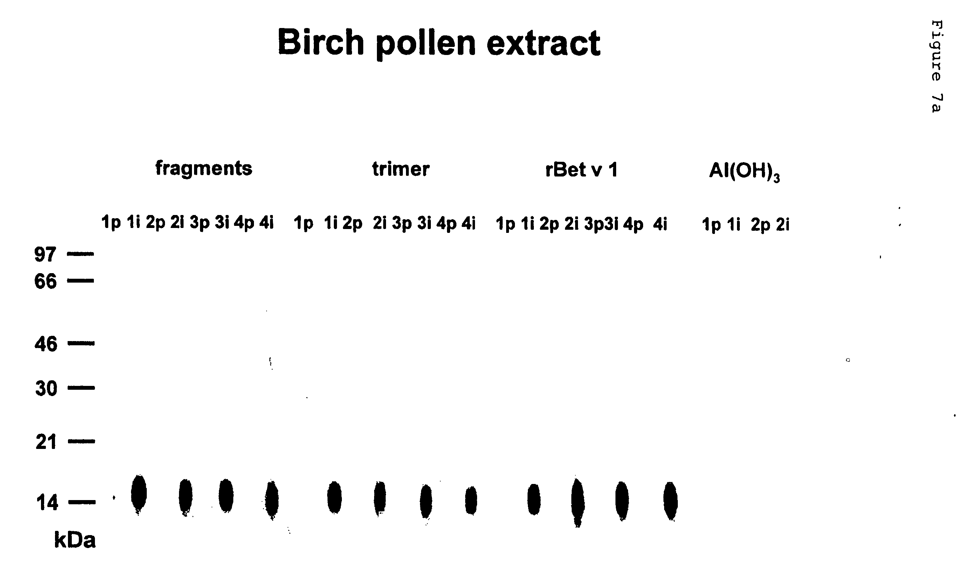

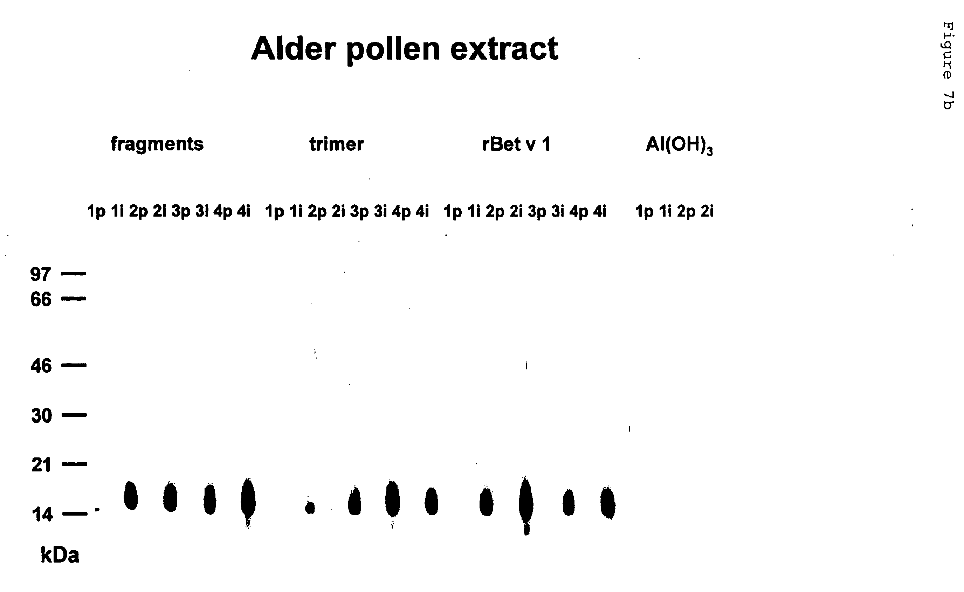

Fig. 6: Induction of IgE responses to rBet v 1 in the different mouse groups. Mean OD values corresponding to Bet v 1-specific IgE level (y-axis) are displayed for the mouse groups treated by the increasing and high dose scheme for the different bleedings (x-axis).

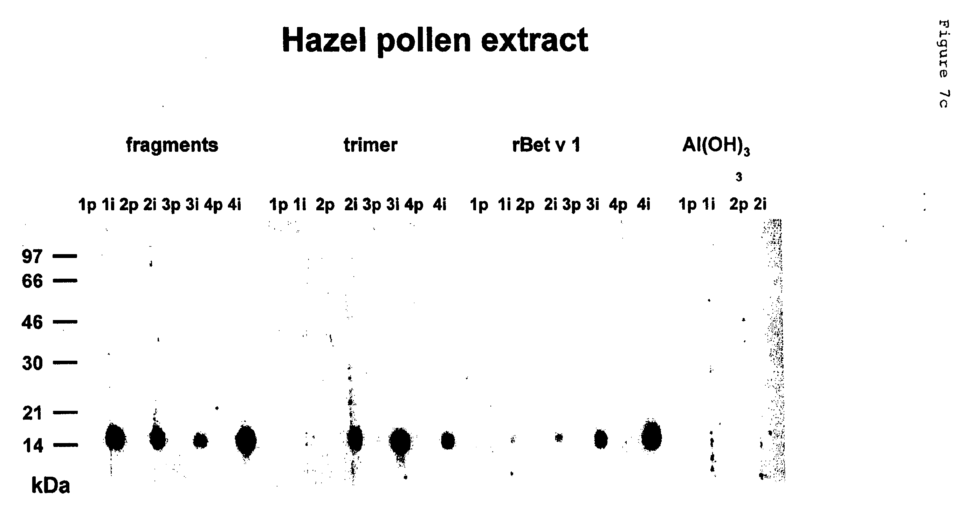

Fig. 7: IgG₁ reactivity of mice to nitrocellulose-blotted pollen extracts. Sera (p: preimmune sera; i: immune sera) from mice immunized with rBet v 1 fragments, trimer, rBet v 1 wildtype, or Al(OH)₃ alone were exposed to nitrocellulose-blotted birch pollen (Figure 7a), alder pollen (Fig. 7b), and hazel pollen (Fig. 7c) extract. Molecular masses are displayed in kDa at the left side.

The following examples further illustrate the invention.

### Example 1: Characterization of recombinant allergen adsorbates

Recombinant rBet v 1 fragments, trimer and rBet v 1 wildtype, as well as Al(OH)₃ adsorbates of the proteins, were assessed for stability at three different temperatures. SDS-PAGE revealed no evidence of degradation of the individual proteins during the course of the study (Figure 2). In the case of the protein adsorbates, no protein could be detected in the supernatants of centrifuged samples of the aluminum hydroxide suspensions using the Lowry method during (data not shown). The pH of the fragments was in the range between 7.9-8.2 and that of the trimer adsorbates between 8.0-8.3.

Repeated dose toxicity studies revealed no evidence for histopathological alterations induced by the allergen adsorbates at the injection site or systemically other than those induced by the adjuvant alone. Comparing the haematological parameters in the different groups no significant differences were found between the groups receiving active preparations or adjuvant alone.

### Experimental protocol:

### Production of adsorbates containing recombinant antigens:

Recombinant Bet v 1a was expressed in *E*. *coli* and purified as described (Hoffmann-Sommergruber, et al., Protein Expr. Purif. 1997, 9: 33-39). Recombinant Bet v 1 fragments, F1 and F2, comprising aa 1-73 (MW 8,100 Dalton) and aa 74-159 (MW 9,461 Dalton) of Bet v 1, respectively, were expressed in *E*. *coli* and purified (Vrtala, S. et al., J. Clin. Invest. 1997, 99: 1673-1681). A stable recombinant trimer consisting of three covalently-linked copies of Bet v 1a was produced by ligation of three copies of the Bet v 1a-encoding cDNA into plasmid pET-17b and expression of the recombinant trimer in *E*. *coli* BL21 (DE3) (Vrtala, S. et al., Int. Arch. Allergy Immunol. 1999, 118: 218-219).

Recombinant Bet v 1 and rBet v 1 trimer were dissolved in 144 mM sodium chloride, 10 mM sodium hydrogencarbonate, 0.25%w/v phenol pH 8.0 and mixed with sterile aluminumhydroxide suspension (Superfos Biosector, Copenhagen, Denmark) to achieve final concentrations of 1 mg Al³⁺/ml and 100 µg protein/ml. An adsorbate containing an equimolar mixture of the two fragments was produced by mixing adsorbates prepared with each fragment as described above for rBet v 1 and trimer in the ratio 0.46 F1: 0.54 F2 to achieve equimolar concentrations of the two fragments. The resulting adsorbates contained a final total protein concentration of 100 µg/ml.

### Characterization of adsorbates:

AI(OH)₃ adsorbates of rBet v 1 fragments, trimer and rBet v 1 wildtype were assessed for stability at three different temperatures for extended periods (5°C: 16 weeks; 25°C: 12 weeks; 40°C: 4 weeks). During the storage periods, the protein content in the supernatant of centrifuged samples of the aluminum hydroxide suspensions was determined using the Lowry method (Lowry, O. et al., J. Biol. Chem. 1951, 193: 265-275).

### Example 2: Antibody responses to genetically-modified hypoallergenic rBet v 1 derivatives administered in courses of 9 injections of increasing concentration

To investigate whether vaccines containing genetically-modified rBet v 1 derivatives induced antibody responses to Bet v 1, sera from the differently immunized groups of mice were analyzed for the presence of rBet v 1-specific IgG₁, IgG_{2a/b} and IgE antibodies (Fig. 3a-c). Figures 3a-c display the mean OD values determined for the 16 animals of each group that had received the increasing dose scheme.

Serum samples obtained at the fourth bleeding (day 77) contained the highest levels of Bet v 1-specific IgG₁ antibodies (Fig. 3a). The strongest IgG₁ response was induced by repeated vaccination with the rBet v 1 fragment-mixture and the unmodified rBet v 1 followed by the trimer (Fig. 3a). The mean rBet v 1-specific IgG₁ antibody-responses (optical density values: least squares means) at day 77 were 0.622 (fragments), 0.593 (rBet v 1), 0.411 (trimer), 0.03 (Al(OH)₃). All responses were significantly greater than those to Al(OH)₃ alone (p<0.00001).

Recombinant allergen-derived vaccine formulations also induced IgG_{2a/b} antibodies (Fig. 3b). rBet v 1 fragment-mixture and rBet v 1 wildtype induced higher levels of rBet v 1-specific IgG_{2a/b} than the trimer formulation (Fig. 3b). The mean IgG_{2a/b} antibody-responses (optical density values: least squares means) obtained at the 4^{th} bleeding (day 77) were 0.136 (rBet v 1), 0.103 (fragments), 0.065 (trimer), and 0.049 (Al(OH)₃).

Measurement of the rBet v 1-specific IgE responses showed that all three recombinant allergen-based vaccines induced only low IgE levels (Fig. 3c). The mean rBet v 1-specific IgE antibody responses (least squares means) measured at the 4^{th} bleeding (day 77) were 0.078 (trimer), 0.060 (fragments, rBet v 1) and 0.042 (Al(OH)₃). Fragment- (p=1.0), rBet v 1- (p=1.0) and trimer- (p=0.114) did not induce a significant IgE-induction compared to the adjuvant-immunization.

Next, the immunogenicity of the two rBet v 1 fragments which were present as equimolar mixture in the fragment formulation was compared. Mice which were immunized with the fragment mixture mounted significantly higher IgG₁ responses to fragment 2 than to fragment 1 (p<0.0001) (Fig. 4).

### Experimental protocol for Example 2 and 3:

### Immunization of mice:

144 (72 male, 72 female) eight-week-old BALB/c mice were purchased from Charles River (Kislegg, Germany). The animals were maintained in the animal care unit of the Department of Pathophysiology of the University of Vienna according to the local guidelines for animal welfare. Groups of 16 mice each (8 male, 8 female) received subcutaneous immunizations in the neck either in increasing doses (I) or as a high dose (H) scheme (Fig. 1). Mice of the increasing dose (I)-scheme received increasing doses (0.1, 0.2, 0.4, 0.8, 1.25, 2.5, 5, 10µg) of either Al(OH)₃ adsorbed rBet v 1 fragments 1 and 2, rBet v 1 trimer, or rBet v 1 in weekly intervals (day 0, 7, 14, 21, 28, 35, 42, 49) until the maintainance dose of 10 µg (equivalent to 1/10 of the conceivable human dose) was reached (day 49). A control group received corresponding injections of an Al(OH)₃ suspension. After a 1-month interval, a final injection of the maintainance dose was given at day 70 (Fig. 1). Mice in the high dose (H)-scheme were injected three times in 3-week-intervals (day 0, 21, 42) with the maintenance dose (10 µg) (Fig.1).

### Clinical, laboratory and histological investigations of mice:

The well being of all animals was regularly assessed. Prior to each injection the body weight of the animals was determined and the injection sites were examined for local responses. Blood samples were collected at day 0, 21, 42, and on completing the study at day 77, in order to assess humoral antibody responses. At day 77, mice were sacrificed after bleeding. Liver, heart, kidneys, spleen and lungs from all animals were weighed and histopathology was obtained for liver, spleen, lungs, lymphnodes, thymus, skin from the injections site and remote from the injection sites. Blood samples from day 77 were analyzed for haematocrit, haemoglobin, erythrocyte counts, mean corpuscular volume (MCV), mean corpuscular haemoglobin concentration (MCHC), leukocyte-, neutrophil- and platelet counts.

### ELISA measurement of specific IgE, IgG₁ and IgG_{2a/b}-antibodies:

Mouse antibodies with specificity for rBet v 1 (Fig. 3a-c) and rBet v 1 fragments (Fig. 4) were measured by ELISA. In brief, ELISA plates (Nunc-Maxisorb^{®}, Nunc, Roskilde, Denmark) were coated with 100 ml/well antigen (diluted in PBS to 10µg/ml) overnight at 4°C. Plates were washed twice with 200 ml PBS, 0.05% v/v Tween and incubated with 200 ml blocking solution (PBS, 0.05% v/v Tween, 1% w/v BSA) for 2.5 hours at room temperature. Plates were then exposed to mouse sera (100 ml/well diluted 1:5 for IgE-, 1:1000 for IgG₁-measurements and 1:50 for IgG_{2a/b} in PBS, 0.05% v/v Tween, 0.5% w/v BSA) overnight at 4°C. After five washes with 200 ml PBS, 0.05% v/v Tween, bound mouse IgE, IgG₁ or lgG_{2a/b} antibodies were detected with monoclonal anti-mouse IgE, -IgG₁ or IgG_{2a/b}-antibodies from rat (PharMingen, San Diego, CA), diluted 1:1000 in PBS, 0.05% v/v Tween, 0.5% w/v BSA, overnight at 4°C. Plates were washed five times with 200 ml PBS, 0.05% v/v Tween and bound rat antibodies were detected with a horseradish peroxidase-coupled anti-rat Ig antiserum from sheep (Amersham Pharmacia Biotech Europe GmbH, Vienna, Austria) diluted 1:2000 in PBS, 0.05% v/v Tween, 0.5% w/v BSA and visualized with ABTS substrate (Sigma, St. Louis). The optical densities (OD) corresponding to the amounts of bound antibodies were determined at 405 nm in an ELISA reader (Dynatech MR 7000, Guernsey Channel, Islands). OD values for the individual plates were determined after the same incubation periods. To exclude plate to plate variabilities reference sera were included on each of the different plates. Results are displayed as mean values of OD measurements from sera of all 16 animals of each group for each bleeding (Fig. 3).

### Statistical analysis:

Results are presented as mean values. Statistical significance of differences in antibody-induction (IgG₁, IgG₂, IgE) was assessed within a four-factorial repeated measurement analysis of variance (ANOVA) model unless otherwise stated. Included factors were time of bleeding, type of vaccine, allergen dose used for immunization and gender. For evaluation of immunization schemes (I *versus* H) over vaccines an additional interaction term between vaccine and dose scheme was included. All pairwise comparisons were Bonferroni adjusted to avoid inflation of α-error. An observed difference was deemed statistically significant if the p-value was lower than 0.05.

### Example 3: Antibody responses to courses of three injections with high doses of genetically engineered rBet v 1 derivatives

Comparing Bet v 1-specific IgG₁ and IgG_{2a/b} antibody levels of mice of the increasing dose scheme (comprising a course of nine injections with antigen amounts gradually increasing to 10 µg) with mice of the high dose scheme (consisting of three injections of the maintenance dose of 10 µg antigen) no significant differences were found at the third bleeding (Fig. 3a and 5).

Moreover we found that immunization according to the high dose scheme induced significantly (p<0.00001) lower IgE-levels than the increasing dose scheme at the time of 3^{rd} and 4^{th} bleeding (Fig. 6).

### Example 4: Hypoallergenic rBet v 1 derivatives induce IgG₁ antibodies that crossreact with natural Bet v 1 and Bet v 1-related allergens

Sera from mice treated with rBet v 1 fragments, trimer and rBet v 1 wildtype exhibited IgG₁ reactivity to nitrocellulose-blotted natural Bet v 1 in a birch pollen extract at 17 kDa (Fig. 7a, lanes 1i-4i). Preimmune sera obtained from the same mice, as well as sera from mice treated with the adjuvant alone did not contain Bet v 1-specific IgG₁ (Fig. 7a, lanes 1p-4p; Al(OH)₃). The immune sera were tested for IgG₁ reactivity to nitrocellulose-blotted alder-and hazel pollen extracts and were found to recognize the major alder pollen allergen Aln g 1, at 17 kDa (Fig. 7b) and contained IgG₁ against the major hazel pollen allergen, Cor a 1 (Fig. 7c).

### SDS-PAGE and immunoblotting:

Protein extracts from birch (*Betula verrucosa*), alder (*Alnus glutinosa*), hazel (*Corylus avelana*) pollen (Allergon AB, Välinge, Sweden) were prepared as described (Niederberger, V., et al., J. Allergy Clin. Immunol., 1998, 102: 579-591). Protein contents and quality of the extracts were analyzed by analytical 12.5% SDS-PAGE and Coomassie Blue staining. A Rainbow Marker (Amersham, Buckinghamshire, U.K.) was used as molecular weight standard. Comparable amounts (100µg/cm preparative gel) of each protein extract were blotted onto nitrocellulose membranes (Schleicher & Schuell, Dassel, Germany). Nitrocellulose membranes were blocked twice for 15 minutes and once for 30 minutes in buffer A (50 mM Na phosphate, pH 7.5, 0.5% w/v BSA, 0.5% v/v Tween, 0.05% w/v NaN₃). From each treatment group two representative immune sera (4^{th} bleeding, day 77) and the corresponding preimmune sera were used for the immunoblotting. After blocking, sheets were incubated with mouse sera diluted 1:1000 in buffer A, overnight at 4°C. Membranes were washed in buffer A and incubated with monoclonal anti-mouse IgG₁ antibodies from rat, diluted 1:1000 in buffer A overnight at 4°C. Bound rat antibodies were detected with 1:1000 diluted ¹²⁵I-labeled anti-rat antibodies from sheep (Amersham Pharmacia Biotech Europe GmbH, Vienna, Austria) and visualized by autoradiography (X-OMAT film, Kodak, Heidelberg, Germany).

### Example 5: Genetically engineered hypoallergenic rBet v 1 derivatives induce antibodies that inhibit binding of IgE from allergic patients' to rBet v 1 wildtype

A competitive ELISA assay was established to investigate, whether murine antibodies induced by vaccination with hypoallergenic rBetv 1 derivatives can inhibit the binding of serum IgE from 10 different birch pollen allergic patients to rBet v 1 wildtype. Two representative serum samples (one male, one female) from each mouse group obtained at the 4^{th} bleeding, together with the corresponding preimmune sera as controls were analyzed for their ability to block human IgE binding to rBet v 1.

Despite the fact that the mouse sera were diluted 1:100 for the competition experiments, they potently blocked IgE binding to Bet v 1. Sera from rBet v 1 fragment-immunized mice (I-scheme) inhibited patients' IgE binding between 58.36 and 89.38% (mean 74.69%) (Table 1). Preincubation with sera from trimer-immunized mice (I-scheme) yielded an inhibition of IgE binding between 14.65 and 85.26% (mean 50.28%) and sera from mice immunized with rBet v 1 wildtype achieved an inhibition of patients' IgE-binding from 32.8 to 84.01% (mean 62.89%). A substantial inhibition of IgE binding to rBet v 1 was also obtained with sera from mice immunized according to the high dose scheme (fragments: 18.18-59.53%, mean 45.28%; trimer: 11.63-46.66%, mean 27.45%; wildtype: 0-52.93%, mean 24.96%) (Table 1).

### Experimental protocol:

### Competitive ELISA for assessment of inhibition of patients' IgE binding to rBet v 1 by murine sera:

ELISA plates (Nunc-Maxisorb^{®}, Nunc, Roskilde, Denmark) were coated with 100 µl/well of 0.5µg rBet v 1/ml, overnight at 4°C. After five TBST washes, plates were blocked with TBST, 1% w/v BSA for 2 hours at 37°C. Then, plates were incubated with mouse sera from each treatment group (sera from the 4^{th} bleeding, day 77 and corresponding preimmune sera, day 0) each diluted 1:100 in TBST, 0.5% w/v BSA. After five TBST washes, plates were incubated with human sera from birch pollen allergic patients diluted 1:5 in TBST, 0.5% w/v BSA overnight at 4°C. After five TBST washes, bound human IgE was detected with alkaline phosphatase (AP)-coupled mouse monoclonal anti-human-IgE antibodies (PharMingen, San Diego, CA) diluted 1:1000 in TBST, 0.5% w/v BSA was incubated at 37°C, then at 4°C for one hour. After further five washes with TBST the ELISA-substrate (Sigma, St. Louis) was added and the OD of the color reaction was read at 405 nm after 15 minutes. All measurements were performed as duplicates.

The percentage of inhibition of IgE after preincubation with immune sera was calculated as follows: %inhibition = 100-(ODᵢₘₘᵤₙₑx100)/ODₚᵣₑᵢₘₘᵤₙₑ. Dₚᵣₑᵢₘₘᵤₙₑ and ODᵢₘₘᵤₙₑ represent the optical density after preincubation with murine preimmune and immune sera, respectively (Denepoux, S. et al., FEBS Lett. 2000, 465: 39-46).

### Characterization of birch pollen allergic patients:

Patients with birch pollen allergy (n=10) were characterized by a case history indicative of birch pollen allergy, positive skin prick test results, RAST and IgE-immunoblotting. Immunoblots were performed as described (Niederberger, V., et al., J. Allergy Clin. Immunol. 1998, 102: 579-591, Kazemi-Shirazi, L. et al., J. Allergy Clin. Immunol. 2000, 105: 116-125). Two patients (P1, P2) exhibited CAP RAST FEIA (Pharmacia, Uppsala, Sweden) class 6, four patients (P3-6) class 5 and four patients (P7-10) class 4.

**Table 1. Inhibition of patients IgE binding to rBet v 1 by murine antisera**

| | | Percentage inhibition of patients' (P1-P10) IgE binding to rBet v 1 | | | | | | | | | | mean inhibition |
|---|---|---|---|---|---|---|---|---|---|---|---|---|
| Murine antiserum raised against: | | P1 | P2 | P3 | P4 | P5 | P6 | P7 | P8 | P9 | P10 | mean |
| increasing dose scheme: | fragments (m) | 75.05 | 89.38 | 79.97 | 81.32 | 76.31 | 86.66 | 65.66 | 65.27 | 60.52 | 80.60 | 76.07 |
| | fragments (f) | 72.33 | 87.00 | 79.89 | 78.04 | 67.74 | 73.57 | 63.30 | 76.32 | 58.36 | 76.65 | 73.32 |
| | trimer (m) | 39.54 | 35.83 | 36.80 | 35.25 | 28.93 | 33.04 | 31.62 | 35.95 | 14.65 | 21.59 | 31.32 |
| | trimer (f) | 67.58 | 85.26 | 72.47 | 72.30 | 65.59 | 80.79 | 56.77 | 70.00 | 50.00 | 71.66 | 69.24 |
| | rBet v 1 (m) | 63.24 | 75.10 | 59.30 | 44.66 | 61.45 | 69.31 | 42.56 | 58.01 | 42.65 | 62.41 | 57.87 |
| | rBet v 1 (f) | 75.18 | 84.01 | 76.88 | 73.16 | 73.20 | 79.64 | 32.80 | 61.38 | 47.03 | 75.88 | 67.92 |
| | | | | | | | | | | | | |
| high dose scheme: | fragments (m) | 57.14 | 59.53 | 53.56 | 51.87 | 45.81 | 55.65 | 39.56 | 52.60 | 24.24 | 18.18 | 45.81 |
| | fragments (f) | 49.93 | 56.81 | 49.70 | 51.89 | 40.34 | 51.99 | 31.01 | 43.41 | 29.90 | 42.58 | 44.76 |
| | trimer (m) | 21.08 | 33.46 | 20.93 | 27.65 | 20.64 | 28.05 | 11.63 | 26.50 | 13.23 | 17.99 | 22.12 |
| | trimer (f) | 38.44 | 46.66 | 35.79 | 41.86 | 24.85 | 42.22 | 14.63 | 31.84 | 20.53 | 31.02 | 32.78 |
| | rBet v 1 (m) | 15.37 | 21.16 | 13.59 | 7.91 | 6.12 | 25.40 | -10.28 | 15.24 | 23.30 | 38.07 | 15.59 |
| | rBet v 1 (f) | 41.77 | 52.93 | 46.70 | 26.55 | 22.91 | 28.72 | 17.76 | 24.46 | 33.71 | 47.87 | 34.34 |

## Claims

1. The use of a derivative of Bet v 1, wherein said derivative is a fragment or an oligomer of Bet v 1 and has a reduced allergenic activity compared with Bet v 1 for the preparation of a medicament for the treatment or prevention of an allergic disorder, wherein the medicament contains Al(OH)₃ and is administered to a patient four times, **characterized in that** the time intervals between the administrations are at least 14 days, and the time interval between the third and the fourth administration being longer than the time intervals between the first three administrations.

2. The use according to claim 1, wherein during each administration substantially the same dose of the derivative is administered.

3. The use according to claim 1 or 2, wherein during each administration a dose of at least 5 µg of the derivative is administered.

4. The use of claim 3, wherein during each administration a dose of at least 100 µg of the derivative is administered.

5. The use according to any of claims 1 to 4 wherein the allergenic activity of the derivative is less than 50% of the allergenic activity of the allergenic protein from which it is derived.

## Patentansprüche

1. Verwendung eines Derivats von Bet v 1, wobei das Derivat ein Fragment oder ein Oligomer von Bet v 1 ist und im Vergleich zu Bet v 1 eine verminderte allergene Aktivität hat, zur Herstellung eines Medikaments zur Behandlung oder Verhütung eines allergischen Leidens, wobei das Medikament Al(OH)₃ enthält und an einen Patienten viermal verabreicht wird, **dadurch gekennzeichnet, dass** die Zeitintervalle zwischen den Verabreichungen mindestens 14 Tage sind und das Zeitintervall zwischen der dritten und vierten Verabreichung länger ist als die Zeitintervalle zwischen den ersten drei Verabreichungen.

2. Verwendung nach Anspruch 1, wobei während jeder Verabreichung im Wesentlichen die gleiche Dosis des Derivats verabreicht wird.

3. Verwendung nach Anspruch 1 oder Anspruch 2, wobei während jeder Verabreichung eine Dosis von mindestens 5 µg des Derivats verabreicht wird.

4. Verwendung nach Anspruch 3, wobei während jeder Verabreichung eine Dosis von mindestens 100 µg des Derivats verabreicht wird.

5. Verwendung nach einem der Ansprüche 1 bis 4, wobei die allergene Aktivität des Derivats weniger als 50% der allergenen Aktivität des allergenen Proteins, aus dem es abgeleitet ist, beträgt.

## Revendications

1. Utilisation d'un dérivé de Bet v 1, dans laquelle ledit dérivé est un fragment ou un oligomère de Bet v 1 et a une activité allergène réduite comparativement à Bet v 1 pour la préparation d'un médicament pour le traitement ou la prévention d'une affection allergique, dans laquelle le médicament contient de l'Al(OH)₃ et est administré à un patient quatre fois, **caractérisée en ce que** les intervalles de temps entre les administrations sont d'au moins 14 jours, et l'intervalle de temps entre la troisième et la quatrième administration étant plus long que les intervalles de temps entre les trois premières administrations.

2. Utilisation suivant la revendication 1, dans laquelle au cours de chaque administration sensiblement la même dose du dérivé est administrée.

3. Utilisation suivant l'une ou l'autre des revendications 1 et 2, dans laquelle au cours de chaque administration une dose d'au moins 5 µg du dérivé est administrée.

4. Utilisation suivant la revendication 3, dans laquelle au cours de chaque administration une dose d'au moins 100 µg du dérivé est administrée.

5. Utilisation suivant l'une quelconque des revendications 1 à 4, dans laquelle l'activité allergène du dérivé est de moins de 50 % de l'activité allergène de la protéine allergène dont il provient.
